Europäisches Patentamt

European Patent Office

Office européen des brevets

(19)

(11) Publication number: **0 170 115 B1**

(12) **EUROPEAN PATENT SPECIFICATION**

(45) Date of publication of patent specification: **05.06.91**   (51) Int. Cl.⁵ **A61K 37/54**, C12N 9/50

(21) Application number: **85108526.6**

(22) Date of filing: **09.07.85**

(54) Thrombus dissolvent.

(30) Priority: **20.07.84 JP 150667/84**

(43) Date of publication of application:
**05.02.86 Bulletin 86/06**

(45) Publication of the grant of the patent:
**05.06.91 Bulletin 91/23**

(84) Designated Contracting States:
**DE FR GB SE**

(56) References cited:
**FR-A- 2 233 038**

**CHEMICAL ABSTRACTS, vol. 92, no. 14, 7th
April 1980, page 354, no. 116420r, Columbus,
Ohio, US; & JP - A - 79 119 017 (TAIYO
FISHERY CO. LTD.) 14-09-1979**

**CHEMICAL ABSTRACTS, vol. 92, no. 14, 7th
April 1980, page 354, no. 116421s, Columbus,
Ohio, US; & JP - A - 79 119 011 (TAIYO
FISHERY CO. LTD.) 14-09-1979**

(73) Proprietor: **Kao Corporation
14-10, Nihonbashi Kayabacho 1-chome
Chuo-Ku Tokyo 103(JP)**

(72) Inventor: **Hara, Kenji
1022-53, Himuromachi
Utsunomiya-shi Tochigi-ken(JP)**
Inventor: **Mansho, Kenji
816-33, Shimoguri-cho
Utsunomiya-shi Tochigi-ken(JP)**

(74) Representative: **Wächtershäuser, Günter, Dr.
Tal 29
W-8000 München 2(DE)**

EP 0 170 115 B1

Note: Within nine months from the publication of the mention of the grant of the European patent. any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

# EP 0 170 115 B1

**Description**

This invention relates to the use of an aqueous extract of krill for the manufacture of a thrombus dissolvent.

Thrombus has relations with various diseases including peripheral artery and vein thrombosis, pulmonary embolism, cardic infarction, coronary thrombosis, cerebral thrombosis, retinal artery and vein thrombosis, presenting serious problems as a pathogenic factor.

For the treatment of thrombosis, it is ordinary to effect a fibrinolytic therapy using urokinase.

However, urokinase has a number of problems: no efficacy appears through oral administration, but the manner of administration is limited only to an intravenous drip; it is very liable to deactivate because of the short half-life period; limitation is placed on the starting material since urokinase is obtained from urine of healthy adult men; administration in large amounts is essential because the half life in blood is short and affinity for fibrin is low; and the administration in large amounts involves increasing tendency toward bleeding. Accordingly, there is a high demand for development of a novel type of thrombus dissolvent in place of urokinase.

Under these circumstance, we made intensive studies to develop a thrombus dissolvent which can be perorally administered and places no limitation on starting materials. As a result, it was found that an extract from krill had the action of dissolving thrombus. The present invention is accomplished on the basis of the above finding.

The present invention provides the manufacture of a thrombus dissolvent which uses an effective amount of an aqueous extract of krill.

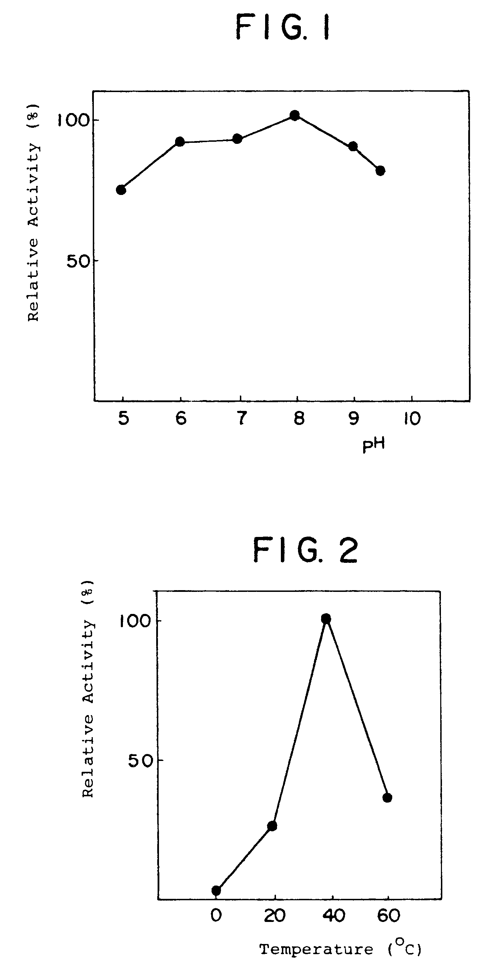

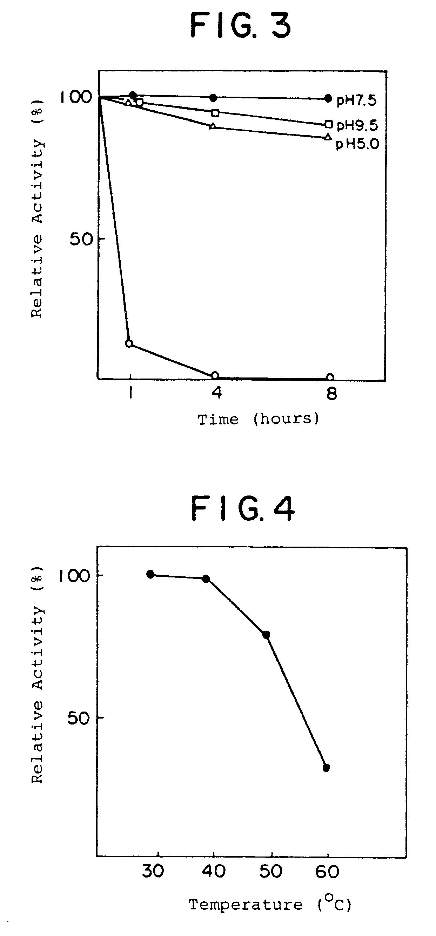

Fig. 1 is a graph showing the pH dependence of the activity of the krill protease obtained in Example 1; Fig. 2 is a similar graph showing the temperature dependence of the activity; Fig. 3 is a graph showing the relation between protease activity and time for different pHs; and Fig. 4 is a graph showing the relation between protease activity and temperature.

## DETAILED DESCRIPTION OF THE INVENTION AND PREFERRED EMBODIMENTS

Krill are distributed in the seas in all parts of the world and live greatly around the Antarctic Ocean. It is estimated that the total amount of krill reaches hundreds of millions to two billions tons. A catch of 50,000,000 to 70,000,000 tons/year, which is comparable to the current total catch all over the world, is considered possible. Thus, stable supply as the starting material is ensured.

The krill used in the present invention are not limited to specific types and may be, for example, those belonging to the genus Euphausia, e.g. superba, crystallorophias, frigida, triacantha, vellantini, lougirostris, lucens, similis, spinifera and recurva, and those belonging to the genus Thysanoessa, e.g. macurura, vicina and gregaria.

The aqueous extract of krill is obtained by mixing krill or broken pieces thereof with water. This aqueous extract has high protease activity, and the protease isolated from the aqueous extract by ordinary enzyme isolation and purification techniques exhibits higher thrombus-dissolving activity.

The isolation and purification of protease (hereinafter referred sometimes to as krill protease) from the aqueous extract is not critical, but is carried out by any known techniques. For instance, there are a number of methods including a method in which broken pieces of krill are mixed with water and allowed to stand, and the supernatant liquid is collected, to which a salt such as ammonium sulfate is added for salting-out. Another method comprises adding an organic solvent such as alcohols or acetone, permitting protease to precipitate. Moreover, there is known a method which comprises concentrating the aqueous extract through ultrafiltration (using, for example, Diaflow Membrane YC, by Amicon Co., Ltd.) and freeze-drying the concentrate. Protease has been isolated by the above methods, followed by fractional purification by an ion-exchange chromatography using diethyl aminoethyl (DEAE)-cellulose and/or DEAE-Sephadex, or by a gel filtration using Sephadex G-50. If isolation is effected using salting-out, protease may be obtained as powder by desalting and freeze-drying after filtration or centrifugal separation. The desalting may be carried out by dialysis or gel filtration using, for example, Sephadex G-25.

The manner of administration of the aqueous krill extract is not critical, but when it is administered orally, very good thrombus-dissolving activity is exhibited.

The amount of the extract is favorably in the range of from 10 to 500 mg/day, calculated as protease, when it is administered orally. However, the amount may be increased depending on the degree of thrombus.

The toxicity of protease which is an aqueous extract of krill is as follows: $LD_{50}$ for rats exceeds 5 g/kg in the case of intraperitoneal administration and 25 g/kg in the case of oral administration.

2

The mechanism of the thrombus-dissolving action of the aqueous krill extract of the invention is not known at the present stage. In view of the fact that the protease contained in the aqueous extract exhibits high activity, it is assumed that the protease serves to dissolve thrombus.

As will be understood from the above, the aqueous krill extract, particularly, protease derived from krill exhibits good thrombus dissolving activity when applied through peroral administration, and is thus a very good thrombus dissolvent which is low in toxicity and involves no side effects.

The present invention is more particularly described by way of examples. In Example 1, protease activity was measured according to the following method.

**[Measurement of Protease Activity]**

One gram of milk casein was suspended in 100 ml of 0.1M phosphate buffer solution (pH 7.6) and boiled for 15 minutes for complete dissolution, thereby obtaining a substrate solution. 1 ml of the substrate solution was added to 4 ml of the buffer solution. 0.1 ml of a sample was added to the obtained solution, followed by reaction at 30°C for 60 minutes. The reaction was stopped by addition of 1 ml of a 12% trichloroacetic acid solution, followed by allowing to stand at room temperature over 30 minutes. The solution was centrifuged under conditions of 10°C, 14000 x g and 20 minutes, and the resulting supernatant liquid was subjected to measurement of its absorption at 280 nm. An activity, which could increase an absorbance by 1 at 280 nm in 10 minutes, was determined as one activity unit.

**Example 1**

Frozen krill (Euphausia superba) were broken into fine pieces, and 400 g of the pieces was added to 1.5 liters of 0.1M phosphate buffer solution (pH 7.0), followed milling at 0°C for 2 minutes by the use of a homogenizer. The resulting homogenate was centrifuged under conditions of 0°C, 6800 x g for 30 minutes to obtain a supernatant liquid.

Ammonium sulfate was added to the supernatant liquid to an extent of 65% saturation, followed by allowing to stand at 0°C for 1 hour and centrifuging to obtain a salted-out, precipitation fraction. Subsequently, the fraction was dispersed in 0.1M phosphate buffer solution having a pH of 7.5 and was dialyzed against distilled water by the use of a cellophane tube and desalted at 4°C, followed by freeze-drying to obtain 7.1 g of protease. The protease had an activity of 335 U/g. The physiological properties of the krill protease are as follows.

(1) Optimum pH

The pH-activity curve of the krill protease was determined using milk casein as the substrate. The results are shown in Fig. 1. The pH buffer solutions used were buffer solutions of 100 mM acetic acid (pH 4.0 - 6.0), phosphoric acid (pH 6.5 - 8.0), and trishydrochloric acid (pH 7.5 - 9.5), which were properly used depending on the desired pH. The krill protease showed high activity in a fairly wide range of pH of from 6.0 - 9.0. Although the pH specificity is wide, an optimum pH is considered to be around 8.

(2) Optimum Temperature

The influence of temperature on the krill protease activity was determined by reaction at different temperatures (0 to 60°C) for 60 minutes at a pH of 7.6 using milk casein as the substrate. The results are shown in Fig. 2, revealing that the optimum temperature was approximately 40°C.

(3) pH Stability

The krill protease was subjected to pre-incubation at a pH of 3 - 10 at 30°C to determine the pH stability. The results are shown in Fig. 3. The krill protease was stable under weakly acidic to weakly alkaline conditions, but was unstable more acidic or more alkaline conditions.

(4) Temperature Stability

The krill protease was allowed to stand at a predetermined temperature for 30 minutes in 0.1 M phosphate buffer solution (pH 7.6) and its activity was measured using milk casein as the substrate to determine the temperature stability. The results are shown in Fig. 4. The protease was confirmed to be deactivated at temperatures over 60° C.

**Example 2**

Fibrinolytic Activity of Krill Protease:

(i) Fibrin Plate Method

The fibrinolytic activity of the protease (65% saturated ammonium sulfate fraction) obtained in Example 1 was measured according to the fibrin plate method.

The fibrin plate was made by a method in which bovine fibrinogen (Sigma Co., Ltd.) was dissolved in a 1/15 M phosphate buffer solution with a pH of 7.5 in a concentration of 0.2%, and 8 ml of the solution was charged into a Petri dish having a diameter of 90 mm, followed by dropping 0.1 ml of a thrombin solution (50 U/ml, by Mochida Seiyaku K.K.) and immediately shaking horizontally. Thereafter, 30 microliters of an enzyme solution (containing 100 micrograms of the protease) was applied onto the fibrin plate, followed by keeping the plate at 37° C for 30 minutes. As a result, fibrinolysis was apparently recognized. The results of measurement of the area of the fibrinolytic window are shown in Table 1. For control, a physiological saline solution was used instead of the enzyme solution.

**Table 1**

| Sample | Protease (in 30 micro-liters) | Area of Fibrino-lytic window |
|---|---|---|
| **Product of Invention:** Protease obtained in Example 1 (ammonium sulfate fraction) | 100 μg | 249 mm$^2$ |
| **Control:** Physiological saline solution | - | 0 |

(ii) Fibrinolysis-promoting Action Through Oral Administration to Rats

Protease (65% saturated ammonium sulfate fraction obtained in Example 1) was orally administered through probes to groups of rats (7 weeks-old, male Wistar rats), each consisting of 10 rats, in an amount of 1 g/kg. Ninety minutes after the oral administration, blood was sampled from aorta abdominals under etherization.

For comparison, physiological saline solution and urokinase (2000 IU/kg), instead of protease, were administered to rat groups, followed by repeating the blood sampling in the same manner as described above.

The fibrinolysis-promoting action was assessed on the basis of the euglobulin dissolution test. In the respective groups, the weight of the fibrin-dissolved solution was measured, with the results shown in Table 2 below.

## Table 2

| Tested Group | Amount of Dissolved Fibrin (g) (average of 10 rats) | Fibrinolysis-promoting Ability in case where control is taken as 100 |
|---|---|---|
| Group (control) administered with physiological saline solution | 0.1973 | 100 |
| Group administered with urokinase | 0.1782 | 90 |
| Group administered with protease* | 0.2604 | 132 |

\* Ammonium sulfate fraction obtained in Example 1.

As will be shown in Table 2, the group administered with protease is improved in fibrinolysis-promoting action by 30% or higher over the control group. Even when orally administered, the krill protease shows the fibrinolytic action as is different from urokinase.

## Claims

1. Use of an aqueous extract of krill for the manufacture of a thrombus dissolvent.

2. Use according to Claim 1 wherein said aqueous extract is protease.

## Revendications

1. Utilisation d'un extrait aqueux de krill pour la fabrication d'un dissolvant de thrombus.

2. Utilisation selon la revendication 1, dans laquelle ledit extrait aqueux est la protéase.

## Ansprüche

1. Verwendung eines wässrigen Extrakts von Krill für die Herstellung eines Mittels zur Thrombusauflösung.

2. Verwendung gemäß Anspruch 1, wobei der wässrige Extrakt Protease ist.

# F I G. I

# F I G. 2

# FIG. 3

# FIG. 4